# EUROPÄISCHE PATENTANMELDUNG

(11) **EP 2 191 861 A1**
(43) Veröffentlichungstag der Anmeldung: **02.06.2010**
(21) Anmeldenummer: 09178736.6
(22) Anmeldetag: 14.03.2002
(51) Int. Cl.: A61M 16/06

(54) **Atemmaskenanordnung sowie Stirnauflageeinrichtung hierzu**

(30) Priorität: 17.01.2002 DE 10201682
(62) Teilanmeldung aus: 02714190.2
(71) Anmelder: MAP Medizin-Technologie GmbH, 82152 Martinsried (DE)
(72) Erfinder: Lang, Bernd, 82166 Gräfelfing (DE); Biener, Achim, 85445 Aufkirchen (DE); Heidmann, Dieter, Castle Hill, NSW 2154 (AU); Vögele, Harald, 82131 Gauting (DE); Madaus, Stefan, 82166 Gräfelfing (DE)
(74) Vertreter: Vossius & Partner

(57) **Zusammenfassung**

Die Erfindung betrifft eine Atemmaskenanordnung zur Zufuhr eines Atemgases zu einem Patienten. Weiterhin betrifft die Erfindung auch eine Stimauflageeinrichtung (10) hierzu. Die erfindungsgemäße Atemmaskenanordnung umfasst einen Gewölbekörper (2), eine Dichtlippeneinrichtung (1) zur Auflage auf der Gesichtsfläche eines Maskenanwenders, eine Atemgasleitungseinrichtung zur Zuleitung von Atemgas zu einem von dem Gewölbekörper begrenzten und mit der Nasen- und/oder Mundöffnung des Maskenanwenders in Verbindung stehenden Maskeninnenraum. Die erfindungsgemäße Atemmaskenanordnung zeichnet sich aus, durch eine Applikationsstruktur (3) zur Applikation der Dichtlippeneinrichtung gemeinsam mit dem Gewölbekörper, wobei die Applikationsstruktur einen Trägerabschnitt (7) aufweist an welchem ein, als Dockingport (8) ausgebildetes Atemgasleitungsorgan, lösbar angebracht ist. Die erfindungsgemäße Stimauflageeinrichtung umfasst ein in Applikationsposition der Atemmaske zur Auflage auf dem Stirnbereich eines Maskenanwenders vorgesehenes Auflageelement (14) sowie eine Halteeinrichtung (12) zur Halterung des Auflageelements in kippbewegbarer Weise. Dadurch wird selbst bei einem vergleichsweise großflächig ausgebildeten Auflageelement eine gute Anpassbarkeit an die unterschiedlichsten Gesichtstekturen erreicht.

## Beschreibung

### Gebiet der Erfindung

Die Erfindung betrifft eine Atemmaskenanordnung wie sie beispielsweise im Rahmen einer CPAP-Therapie zur Behandlung schlafbezogener Atmungsstörungen Anwendung finden kann. Weiterhin betrifft die Erfindung auch eine Stirnauflageeinrichtung für eine Atemmaskenanordnung.

### Hintergrund der Erfindung

Im Rahmen der genannten CPAP-Therapie kann einem Patienten über eine Atemmaskenanordnung ein atembares Gas, insbesondere Umgebungsluft auf einem Druckniveau zugeführt werden das über dem Umgebungsdruckniveau liegt. Durch das unter Druck stehende Atemgas kann eine pneumatische Schienung der oberen Atemwege erreicht werden und hierdurch etwaigen Obstruktionen vorgebeugt werden. Im Rahmen der Durchführung einer Druckbeatmungs- bzw. CPAP-Therapie werden die zur Zufuhr des Atemgases erforderlichen Atemmaskenanordnungen üblicherweise über die gesamte Schlaf- oder Ruhephase des Patienten hinweg von diesem getragen. Die Atemmaskenanordnung stützt sich üblicherweise über eine Dichtlippenzone im Umgebungsbereich der Nase des Maskenanwenders sowie über eine Stirnauflageeinrichtung im Stirnbereich des Maskenanwenders ab. Die zur Applikation der Atemmaskenanordnung erforderlichen Haltekräfte können durch eine Fixiereinrichtung die zum Beispiel ein um den Hinterkopf des Maskenanwenders herumgeführtes Kopfband aufweist, aufgebracht werden. Im Bereich der Auflagezone der Dichtlippeneinrichtung sowie im Auflagebereich der Stirnauflageeinrichtung können unter Umständen Flächenpressungen auftreten, die zu einer erheblichen Beeinträchtigung des Tragekomforts der Atemmaskenanordnung führten. In Abhängigkeit von der individuellen Gesichtstektur des Maskenanwenders sind teilweise erhebliche Maskenanpreßkräfte erforderlich, um die gewünschte Dichtwirkung zu erreichen. Im Bereich der Auflagezonen der Atemmaske auf dem Gesicht des Patienten können hierbei in unangenehmer Weise deutlich sichtbare Druckstellen auch im Stirnbereich verursacht werden.

### Aufgabe der Erfindung

Der Erfindung liegt die Aufgabe zugrunde, eine Atemmaskenanordnung zu schaffen, die zuverlässig in einer korrekten Applikationsposition fixiert werden kann und sich zudem durch einen hohen Tragekomfort auszeichnet.

### Erfindungsgemäße Lösung

Diese Aufgabe wird erfindungsgemäß gelöst, durch eine Atemmaskenanordnung mit einem Gewölbekörper, einer Dichtlippeneinrichtung zur Auflage auf der Gesichtsfläche eines Maskenanwenders, einer Atemgasleitungseinrichtung zur Zuleitung von Atemgas zu einem von dem Gewölbekörper begrenzten und mit der Nasen- und/oder Mundöffnung des Maskenanwenders in Verbindung stehendem Maskeninnenraum und einer Applikätionsstruktur zur Applikation der Dichtlippeneinrichtung gemeinsam mit dem Gewölbekörper, wobei die Applikationsstruktur einen Trägerabschnitt aufweist an welchem ein Atemgasleitungsorgan angebracht ist.

Dadurch wird es auf vorteilhafte Weise möglich, eine robust aufgebaute und in vorteilhafter Weise reinigbare Atemmaskenanordnung zu schaffen, die sich durch eine hohe Dichtigkeit auszeichnet.

Gemäß einer besonders bevorzugten Ausführungsform der Erfindung ist das Atemgasleitungsorgan als Rohrstutzen ausgebildet. Ein derartiger Rohrstutzen ist vorzugsweise aus einem spülmaschinenfesten Kunststoffmaterial gefertigt, so dass dieses Atemgasleitungsorgan bei vergleichsweise hohen Temperaturen gereinigt und dabei sterilisiert werden kann. Der Rohrstutzen ist vorzugsweise derart ausgebildet, dass dessen Innendurchmesser im Bereich von 12 bis 34 mm liegt. Der Rohrstutzen kann einen, im wesentlichen kreisförmigen oder vorzugsweise einen polygonalen Querschnitt aufweisen. Der Rohrstutzen kann als Leitungskrümmer ausgebildet sein, durch welchen eine leichte Umlenkung des Atemgasstromes um einen Winkel im Bereich von 0 bis 45° erfolgt.

Das insbesondere als Rohrstutzen ausgebildete Leitungsorgan ist vorzugsweise lösbar an dem Trägerabschnitt anbringbar. Zur Ankoppelung des Atemgasleitungsorganes an den Trägerabschnitt ist vorzugsweise eine Verrastungseinrichtung vorgesehen. Diese Verrastungseinrichtung ist gemäß einem besonderen Aspekt der vorliegenden Erfindung in vorteilhafter Weise als Bajonett- bzw. Drehverrastungseinrichtung ausgebildet.

Die Applikationsstruktur der Atemmaskenanordnung umfasst in vorteilhafter Weise einen Rahmenabschnitt der mit der Dichtlippeneinrichtung und/oder mit dem Gewölbekörper lösbar koppelbar ist. Vorzugsweise ist der Rahmenabschnitt derart ausgebildet, dass dieser den Gewölbekörper ring- oder bügelartig umfasst. Der Trägerabschnitt ist in vorteilhafter Weise aus einem Kunststoffmaterial gefertigt und mit Halteorganen versehen, über welche der Trägerabschnitt beispielsweise mit einer unteren Gurtbandanordnung eines Kopfbandes, koppelbar ist. In vorteilhafter Weise sind diese Halteorgane als Haltebügel ausgebildet, durch welche ein Endabschnitt der genannten unteren Gurtbandanordnung hindurchführbar ist. Diese Haltebügel sind vorzugsweise integral mit dem Trägerabschnitt ausgebildet. Die Innenumfangswandung einer von den Haltebügeln gebildeten Durchführungsöffnung, weist vorzugsweise einen Verlauf auf, der hinsichtlich des Formwerkzeuges eine schieberfreie Entformung der Haltebügel gestattet.

Der zur Fixierung des Atemgasleitungsorganes vorgesehene Trägerabschnitt ist in vorteilhafter Weise integral mit dem Rahmenabschnitt ausgebildet. Der Trägerabschnitt ist hierbei mit Vorteil derart ausgebildet, dass dieser eine Einsatzöffnung aufweist, in die das Atemgasleitungsorgan lösbar einsetzbar ist. Der Trägerabschnitt ist vorzugsweise derart angeordnet, dass dieser im wesentlichen senkrecht, zu einer durch den Rahmenabschnitt definierten Rahmenfläche verläuft.

Im Bereich der Einsatzöffnung bildet der Trägerabschnitt vorzugsweise jene mit dem als Rohrstutzen ausgebildeten Atemgasleitungsorgan in Eingriff bringbare Koppelungsstrukturen.

Der Gewölbekörper weist vorzugsweise einen Koppelungsabschnitt auf, über welchen der Gewölbekörper in abdichtender Weise mit dem Atemgasleitungsorgan verbindbar ist. Vorzugsweise ist der Gewölbekörper aus einem Elastomermaterial gefertigt und unter elastischer Aufweitung auf einen, durch die Einsatzöffnung hindurchgeführten und zur Dichtlippeneinrichtung vordringenden Abschnitt des Rohrstutzens aufgesetzt. An diesem zur Dichtlippeneinrichtung vordringenden Abschnitt des Rohrstutzens ist vorzugsweise ein Umfangswulst ausgebildet, durch welchen der Gewölbekörper und der Rohrstutzen auf zuverlässige Weise in Fügestellung gehalten werden.

Gemäß einer besonders bevorzugten Ausführungsform der Erfindung ist der Gewölbekörper integral mit der Dichtlippeneinrichtung ausgebildet. Dadurch wird es auf vorteilhafte Weise möglich, Fugen bzw. Spalten im Übergangsbereich zwischen Dichtlippeneinrichtung und Gewölbekörper zu vermeiden. Darüber hinaus wird es auf vorteilhafte Weise möglich, die Dichtlippeneinrichtung und den Gewölbekörper als elastomeres Integralteil in den Rahmenabschnitt einzusetzen.

Eine besonders zuverlässige Ableitung von CO₂ befrachtetem Atemgas an die Umgebung wird gemäß einer besonders bevorzugten Ausführungsform der Erfindung dadurch erreicht, dass der Gewölbekörper mit Öffnungen versehen ist, durch welche das im Innenbereich des Gewölbekörpers unter Druck stehende Atemgas zur Umgebung hin entweichen kann. Die Öffnungen sind vorzugsweise derart ausgebildet, dass sich deren Querschnitt in Austrittsöffnung erweitert. Diese Austrittsöffnungen sind vorzugsweise derart angeordnet, dass sich diese möglichst nahe in dem, in Applikationsposition der Atemmaskenanordnung den Nasenöffnungen eines Maskenanwenders benachbarten Bereich befinden.

Gemäß einem besonderen Aspekt der vorliegenden Erfindung umfasst die Applikationsstruktur eine Stirnabstützeinrichtung zur Abstützung der Atemmaskenanordnung im Stirnbereich des Maskenanwenders. Die Stirnabstützeinrichtung ist in vorteilhafter Weise über eine Gelenkvorrichtung mit dem den Gewölbekörper umgreifenden Rahmenabschnitt verbunden. Dadurch wird es auf vorteilhafte Weise möglich, die Position der Stirnabstützeinrichtung an die individuelle Gesichtstektur des Maskenanwenders anzupassen. Vorzugsweise umfasst die Gelenkeinrichtung eine Bogenbahnführung, durch welche die Stirnabstützeinrichtung veränderbar positionierbar ist.

Das vorzugsweise lösbar, mit dem Trägerabschnitt koppelbare Atemgasleitungsorgan, bildet in vorteilhafter Weise einen Dockingport der beispielsweise auch Teil einer Dreh-Koppelungsstruktur bilden kann. An dem Dockingport können weitere Anschlusseinrichtungen, insbesondere kleinere Rohrstutzen ausgebildet sein, über welche beispielsweise ein Druckmessschlauch an die Atemmaskenanordnung angekoppelt werden kann oder gegebenenfalls auch eine zusätzliche Sauerstoffzufuhr erfolgen kann.

Die Stirnabstützeinrichtung ist vorzugsweise aus einem thermoplastischen Kunststoffmaterial gefertigt und mit einer Stimpolstereinrichtung versehen. Die Stimpolstereinrichtung ist vorzugsweise durch pad-artig ausgebildete Elastomerelemente gebildet, die über eine Steckverbindungsstruktur mit dem vorzugsweise bügelartig ausgebildeten Aufnahmeabschnitt der Stirnabstützeinrichtung koppelbar sind. Die Stirnabstützeinrichtung ist dabei vorzugsweise derart ausgebildet, dass die Elastomerelemente an unterschiedlichen Stellen an der Stirnabstützeinrichtung anbringbar sind. Vorzugsweise sind die Elastomerelemente weiterhin derart ausgebildet, dass durch die Art der Befestigung derselben an dem Bügelteil ebenfalls unterschiedliche Positionen der Auflagebereiche der Elastomerelemente auf der Stirn des Maskenanwenders erreichbar sind. Die Elastomerelemente sind vorzugsweise aus einem Silikonkautschukmaterial gefertigt und im Bereich ihrer Auflagefläche derart geformt, dass die Übertragung der Aulagegekräfte auf die Stirnfläche des Maskenanwenders unter einer physiologisch gut verträglichen Flächenpressung erfolgt. Dies kann insbesondere dadurch erreicht werden, dass die Elastomerelemente auf einer ihrer Auflageseite abgewandten Rückseite mit einem exzentrisch angeordneten Standfuß versehen sind, der eine Kippbewegung des am Maskenanwender aufsitzenden Auflageabschnitts zulässt.

An dem Bügelteil sind vorzugsweise auch Koppelungsabschnitte vorgesehen, welche eine Koppelung der Stirnabstützeinrichtung mit einer oberen Stirnbandanordnung eines Kopfbandes ermöglicht. Diese Koppelungsabschnitte können als Bandlasche ausgebildet sein. Es ist auch möglich, die Stirnabstützeinrichtung, beispielsweise über eine Klettverschlusseinrichtung an einer vorzugsweise gepolsterten Stirnbandanordnung zu befestigen.

Gemäß einem weiteren Aspekt der Erfindung liegt dieser auch Aufgabe zugrunde, der Entstehung etwaiger Druckstellen im Stirnbereich im Zusammenhang mit der Anwendung von Atemmasken vorzubeugen.

Diese Aufgabe wird erfindungsgemäß gelöst durch eine Stirnauflageeinrichtung für eine Atemmaske mit einem in Applikationsposition zur Auflage auf dem Stirnbereich eines Maskenanwenders vorgesehenen Auflageelement, wobei eine Halteeinrichtung vorgesehen ist, zur Halterung des Auflageelementes in bewegbarer Weise.

Dadurch wird es auf vorteilhafte Weise möglich, die Abstützung einer Atemmaskenanordnung im Stirnbereich eines Patienten unter deutlich verringerter Gewebe-Flächenpressung zu gewährleisten. Durch die erfindungsgemäß gegebene Bewegbarkeit des Auflageelementes kann sich dieses selbsttätig an die individuelle Krümmung des Stirnbereiches des Maskenanwenders anpassen. Dadurch wird es weiterhin auf vorteilhafte Weise möglich, das Auflagelement großflächig auszubilden, wodurch in vorteilhafter Weise eine deutliche Verringerung der Flächenpressung erreicht werden kann.

Eine gemäß einem besonderen Aspekt der vorliegenden Erfindung bevorzugte Ausführungsform der Stirnauflageeinrichtung ist dadurch gegeben, daß die Halterung als Schwenkhalterung ausgebildet ist. Diese Schwenkhalterung erlaubt vorzugsweise eine Kippbewegung des Auflageelementes, um mindestens eine zur üblichen Auflageausrichtung im wesentlichen parallele Achse. Diese Schwenkhalterung kann vorzugsweise durch eine Gelenkeinrichtung gebildet sein, die gemäß einer besonders bevorzugten Ausführungsform ein Kugelgelenk umfaßt. Alternativ dazu oder auch in Kombination mit dieser Ausgestaltung ist es möglich, die Gelenkeinrichtung durch eine Elastomerstruktur zu bilden. Der Bewegungsbereich der Halterung des Auflageelementes liegt vorzugsweise im Bereich von 10 - 30°. Innerhalb dieses Winkelbereiches kann sämtlichen möglichen Stimtekturen ausreichend Rechnung getragen werden.

Das Auflageelement ist gemäß einem besonderen Aspekt der vorliegenden Erfindung aus einem Elastomermaterial, beispielsweise aus einem volltransparenten oder eingefärbten Silikonkautschukmaterial gebildet. Insbesondere bei dieser Ausführungsform weist das Auflageelement eine vorzugsweise pad- oder tellerartige Gestalt auf. Das Auflageelement ist hierbei vorzugsweise derart konkav einwärts gewölbt, daß sich beim Aufsetzen des Auflageelementes auf die Stirnfläche eine definierte Flächenpressungsverteilung ergibt. Gemäß einer besonders bevorzugten Ausführungsform der Erfindung wird diese Flächenpressung derart gewählt, daß sich innerhalb eines vorbestimmten Abstands vom Rand des Auflageelementes eine weitgehend gleichmäßige Flächenpressung ergibt, wobei im Randbereich des Auflageelements die Flächenpressung nach außen hin allmählich abnimmt.

Gemäß einer besonders bevorzugten Ausführungsform der Erfindung umfaßt die Stirnauflageeinrichtung mehrere, vorzugsweise zwei, einander benachbart angeordnete Auflageelemente. Die Auflageelemente sind vorzugsweise derart angeordnet, daß diese in Applikationsposition einer Atemmaskeneinrichtung oberhalb der linken und rechten Augenbrauen des Maskenanwenders positioniert sind. Die beiden Auflageelemente sind vorzugsweise über eine flexible Stegeinrichtung miteinander verbunden. Hierdurch kann zum einen eine weitere Vergrößerung der Auflagefläche erreicht und zudem die Verdrehmöglichkeit der Auflageelemente zueinander definiert eingeschränkt werden. Insbesondere bei dieser Ausführungsform sind die beiden Auflageelemente vorzugsweise miteinander integral ausgebildet. Die Gestalt der Auflageelemente in einer Draufsicht ist nicht auf im wesentlichen kreisförmige Außenkonturen beschränkt. Beispielsweise ist es auch möglich, elliptische oder andere polygonale Außenkonturen zu wählen.

Die Gelenkeinrichtung zur schwenkbewegbaren Lagerung des jeweiligen Auflageelementes ist vorzugsweise ebenfalls integral mit dem Auflageelement ausgebildet. Ein definierter Gelenkeharakter kann durch entsprechende Geometriegestaltung der Gelenkeinrichtung erreicht werden.

Die Gelenkeinrichtung ist vorzugsweise derart angeordnet, daß sich diese auf oder zumindest nahe einer Kraftwirkungslinie befindet, die durch einen Flächenpressungsschwerpunkt des jeweiligen Auflageelementes verläuft. Hierdurch wird die Vergleichmäßigung der Flächenpressungsverleilung noch weiter unterstützt.

Die Auflageelemente sind vorzugsweise derart profiliert ausgebildet, daß ein Ansaugen des Auflageelementes auf der Stirnfläche des Patienten verhindert wird. Einem Ansaugen der Stirnauflageeinrichtung auf der Stirnfläche des Patienten kann weiterhin auch dadurch vorgebeugt werden, daß das Auflageelement mit Durchgangsbohrungen oder auch mit Kanälen versehen ist, durch welche Luft in einen Zwischenbereich zwischen dem Auflageelement und der Stirn des Maskenanwenders eintreten kann.

Durch die Kugelgelenkstrukturen wird in vorteilhafter Weise eine gute Anpaßbarkeit an den horizontalen sowie an der vertikalen Stirnverlauf des Maskenanwenders erreicht. Die Gelenkeinrichtung - insbesondere die Kugelgelenkeinrichtung - kann auch feststellbar ausgerichtet sein. Die Gelenkeinrichtung kann in weiterhin vorteilhafter Weise auch um bestimmt Achsen - insbesondere um eine Horizontalachse - bevorzugt kippbar sein. Die Wölbung des Auflageelementes kann derart gewählt sein, daß sich in Horizontal- und Vertikalrichtung unterschiedliche Krümmungsradien ergeben. Die Krümmungsradien sind vorzugsweise kleiner gewählt, als übliche Stirnkrümmungsradien.

Alternativ zu Kugelgelenkstrukturen sind auch kardanische Aufhängungen - beispielsweise mittels Gelenkstift möglich. Der Schwenkwinkel der Gelenkeinrichtung ist vorzugsweise auf einen vorgegebenen Anschlagwinkel begrenzt. Die Materialeigenschaften des Auflageelementes sind vorzugsweise derart gewählt, daß sich dieses im wesentlichen antibakteriell und ggf. wundheilungsfördernd verhält.

In vorteilhafter Weise kann im Bereich der Auflagefläche eine Polstereinrichtung, insbesondere eine Gelkörperpolstereinrichtung, oder auch eine Luft- oder Flüssigkeitspolstereinrichtung ausgebildet sein. Durch Variation der eingebrachten Flüssigkeit-, Luft- oder Gelmenge kann hierbei auf vorteilhafte Weise die Position der Atemmaske relativ zum Maskenanwender eingestellt werden.

In vorteilhafter Weise ist die Lagerungsposition der Auflageelemente in einstellbarer Weise veränderbar. Alternativ hierzu - oder auch in Kombination mit dieser Maßnahme ist es auch möglich, mehrere Kopplungsmöglichkeiten vorzusehen, so daß sich verschiedene Stirnabstände durch entsprechende, selektive Koppelung erreichen lassen. Es ist möglich, durch beispielsweise 2, vorzugsweise 3 oder auch mehrere Kopplungsmöglichkeiten eine GrobEinstellung vorzunehmen und innerhalb eines begrenzten Feinjustierbereich eine vorzugsweise stufenlose Feinjustierung vorzunehmen. Es ist möglich mehrere Permutationsmöglichkeiten zu erlauben, wobei die einzelnen Koppelungspermutationen zu jeweils unterschiedlichen Einstellungen bezüglich des Stirnabstandes führen. Es ist auch möglich, Klemmstrukturen vorzusehen durch welche eine stufenlose Einstellung des Stirnabstandes möglich ist. Die Koppelungsstellen können derart ausgebildet sein, daß eine definierte Klebestelle erreicht wird, so daß eine stufenlos individuell angepaßte Einstellung durch Klebung dauerhaft erhalten bleibt.

Ein besonders hoher Tragekomfort wird gemäß einer besonders bevorzugten Ausführungsform der Erfindung dadurch erreicht, daß die mit der Hautfläche des Maskenanwenders in Kontakt gelangenden Oberflächenabschnitte des Auflageelementes eine samtartig matte Oberfläche aufweisen. Gem. einer besonders bevorzugten Ausführungsform der Erfindung ist zumindest im Bereich der Auflagefläche des Auflageelementes eine Oberflächenstruktur zur Erreichung eines Selbstreinigungseffektes vorgesehen. Eine derartige Oberflächenstruktur kann beispielsweise Lotusblatt-Oberflächenstrukturen aufweisen. Das Auflageelement kann auch, zumindest im Bereich der Auflagefläche, mit einem Gelkörper versehen sein.

Die folgenden Ausführungsformen sind bevorzugte Aspekte der vorliegenden Erfindung.
1. Atemmaskenanordnung mit einem Gewölbekörper, einer Dichtlippeneinrichtung zur Auflage auf der Gesichtsfläche eines Maskenanwenders, einer Atemgasleitungseinrichtung zur Zuleitung von Atemgas zu einem von dem Gewölbekörper begrenzten und mit der Nasen- und/oder Mundöffnung des Maskenanwenders in Verbindung stehenden Maskeninnenraum und einer Applikationsstruktur zur Applikation der Dichtlippeneinrichtung gemeinsam mit dem Gewölbekörper, wobei die Applikationsstruktur einen Trägerabschnitt aufweist an welchem ein Atemgasleitungsorgan lösbar angekoppelt ist.
2. Atemmaskenanordnung nach Aspekt 1, **dadurch gekennzeichnet, dass** das Atemgasleitungsorgan als Rohrstutzen ausgebildet ist.
3. Atemmaskenanordnung nach Aspekt 1 oder 2, **dadurch gekennzeichnet, dass** die Applikationsstruktur einen Rahmenabschnitt umfasst.
4. Atemmaskenanordnung nach wenigstens einem der Aspekte 1 bis 3, **dadurch gekennzeichnet, dass** der Rahmenabschnitt integral mit dem Trägerabschnitt ausgebildet ist.
5. Atemmaskenanordnung nach wenigstens einem der Aspekte 1 bis 4, **dadurch gekennzeichnet, dass** der Trägerabschnitt eine Einsatzöffnung aufweist und das Atemgasleitungsorgan lösbar in diese Einsatzüffnung einsetzbar ist.
6. Atemmaskenanordnung nach wenigstens einem der Aspekte 1 bis 5, **dadurch gekennzeichnet, dass** eine Verrastungseinrichtung vorgesehen ist, zur Fixierung des Atemgasleitungsorganes an dem Trägerabschnitt.
7. Atemmaskenanordnung nach wenigstens einem der Aspekte 1 bis 6, **dadurch gekennzeichnet, dass** die Verrastungseinrichtung als Bajonett- bzw. Drehverrastungseinrichtung ausgebildet ist.
8. Atemmaskenanordnung nach wenigstens einem der Aspekte 1 bis 7, **dadurch gekennzeichnet, dass** der Trägerabschnitt im wesentlichen quer zum Rahmenabschnitt ausgerichtet ist.
9. Atemmaskenanordnung nach wenigstens einem der Aspekte 1 bis 8, **dadurch gekennzeichnet, dass** der Gewölbekörper abdichtend mit dem Atemgasleitungsorgan gekoppelt ist.
10. Atemmaskenanordnung nach wenigstens einem der Aspekte 1 bis 9, **dadurch gekennzeichnet, dass** der Gewölbekörper aus einem Elastomermaterial gefertigt ist.
11. Atemmaskenanordnung nach wenigstens einem der Aspekte 1 bis 10, **dadurch gekennzeichnet, dass** der Gewölbekörper integral mit der Dichtlippeneinrichtung ausgebildet ist.
12. Atemmaskenanordnung nach wenigstens einem der Aspekte 1 bis 11, **dadurch gekennzeichnet, dass** der Gewölbekörper mit Öffnungen versehen ist, zur Ableitung von CO₂ befrachtetem Atemgas.
13. Atemmaskenanordnung nach wenigstens einem der Aspekte 1 bis 12, **dadurch gekennzeichnet, dass** die Applikationsstruktur eine Stirnabstützeinrichtung umfasst, zur Abstützung der Anordnung im Stirnbereich.
14. Atemmaskenanordnung nach wenigstens einem der Aspekte 1 bis 13, **dadurch gekennzeichnet, dass** eine Gelenkvorrichtung vorgesehen ist und die Stirnabstützeinrichtung mit dem Rahmenabschnitt (3) schwenkbewegbar gekoppelt ist.
15. Atemmaskenanordnung nach wenigstens einem der Aspekte 1 bis 14, **dadurch gekennzeichnet, dass** die Gelenkeinrichtung eine Bogenbahnführung umfasst und die Stirnabstützeinrichtung einen Führungsabschnitt aufweist.
16. Atemmaskenanordnung mit einem Gewölbekörper, einer Dichtlippeneinrichtung zur Auflage auf der Gesichtsfläche eines Maskenanwenders, einer Atemgasleitungseinrichtung zur Zuleitung von Atemgas zu einem von dem Gewölbekörper begrenzten und mit der Nasen- und/oder Mundöffnung des Maskenanwenders in Verbindung stehenden Maskeninnenraum und einer Applikationsstruktur zur Applikation der Dichtlippeneinrichtung gemeinsam mit dem Gewölbekörper, wobei der Gewölbekörper und die Dichtlippeneinrichtung integral aus einem Elastomermaterial gefertigt und als eine lösbar fixierbare Einheit in die Applikationsstruktur einsetzbar sind.
17. Atemmaskenanordnung nach Aspekt 16, **dadurch gekennzeichnet, das** diese modular aus einem Rahmenabschnitt (3), einer mit dem Rahmenabschnitt (3) lösbar koppelbaren Stirnabstützeinrichtung (10) und einem aus Dichtlippeneinrichtung (1) und Gewölbekörper (2) bestehenden Integralteil zusammensetzbar ist.
18. Stirnauflageeinrichtung für eine Atemmaske mit einem in Applikationsposition zur Auflage auf dem Stirnbereich eines Maskenanwenders vorgesehenen Auflageelement, gekennzeichnet durch eine Halteeinrichtung zur Halterung des Auflageelementes in kippbewegbarer Weise.
19. Stirnauflageeinrichtung nach Aspekt 18, **dadurch gekennzeichnet, daß** die Halterung als Schwenkhalterung ausgebildet ist.
20. Stinauflageeinrichtung nach Aspekt 18 oder 19, **dadurch gekennzeichnet, daß** die Halterung eine Gelenkeinrichtung umfaßt.
21. Stirnauflageeinrichtung nach wenigstens einem der Aspekte 18-20, **dadurch gekennzeichnet, daß** die Gelenkeinrichtung durch eine Elastomerstruktur gebildet ist.
22. Stirnauflageeinrichtung nach wenigstens einem der Aspekte 18 - 21, **dadurch gekennzeichnet, daß** die Gelenkeinrichtung als Kugelgelenkeinrichtung ausgebildet ist.
23. Stirnauflageeinrichtung nach wenigstens einem der Aspekte 18 - 22, **dadurch gekennzeichnet, daß** das Auflageelement aus einem Elastomermaterial gebildet ist.
24. Stirnauflageeinrichtung nach wenigstens einem der Aspekte 18 - 23, **dadurch gekennzeichnet, daß** das Auflageelement padartig ausgebildet ist.
25. Stirnauflageeinrichtung nach wenigstens einem der Aspekte 18-24, **dadurch gekennzeichnet, daß** das Auflageelement eine konkav einwärts gewölbte Auflagefläche bildet, wobei die Wölbung derart abgestimmt ist, daß sich eine definierte Flächenpressungsverteilung ergibt.
26. Stirnauflageeinrichtung nach wenigstens einem der Aspekte 18 - 25, **dadurch gekennzeichnet, daß** zwei Auflageelemente vorgesehen sind.
27. Stirnauflageeinrichtung nach wenigstens einem der Aspekte 18 - 26, **dadurch gekennzeichnet, daß** die zwei Auflageelemente miteinander verbunden sind.
28. Stirnauflageeinrichtung nach wenigstens einem der Aspekte: 18 - 27, **dadurch gekennzeichnet, daß** die Auflageelemente miteinander integral ausgebildet sind.
29. Stirnauflageeinrichtung nach wenigstens einem der Aspekte 18 - 28, **dadurch gekennzeiehnet, daß** die Gelenkeinrichtung integral mit dem Auflageelement ausgebildet ist.
30. Stirnauflageeinrichtung nach wenigstens einem der Aspekte 18 - 29, **dadurch gekennzeichnet, daß** die Gelenkeinrichtung derart angeordnet und ausgebildet ist, daß diese sich auf oder nahe einer Kraftwirkungslinie befindet, die durch einen Flächenpressungsschwerpunkt des entsprechenden Auflageelementes verläuft.
31. Stirnauflageeinrichtung nach wenigstens einem der Aspekte 18 - 30, **dadurch gekennzeichnet, daß** die Auflagefläche derart profiliert ausgebildet ist, daß ein Ansaugen auf der Stirnfläche des Patienten verhindert ist.
32. Stirnauflageeinrichtung nach wenigstens einem der Aspekte 18 - 31, **dadurch gekennzeichnet, daß** das Auflageelement mit Löchern oder Kanälen versehen ist.
33. Stirnauflageeinrichtung nach wenigstens einem der Aspekte 18 - 32, **dadurch gekennzeichnet, daß** zumindest der mit der Hautfläche des Maskenanwenders in Kontakt gelangenden Oberflächenabschnitt des Auflageelementes eine samtartig matte Oberfläche aufweist.
34. Stirnauflageeinrichtung nach wenigstens einem der Aspekte 18 - 33, **dadurch gekennzeichnet, daß** hinsichtlich der Ankoppelung der Stirnauflageeinrichtung mehrere Koppelungsmöglichkeiten vorgesehen sind, so daß durch selektive Koppelung insbesondere Steckverbindung, unterschiedliche Stirnabstände einstellbar sind.

### Kurzbeschreibung der Zeichnungen

Weitere Einzelheiten und Merkmale der Erfindung ergeben sich aus der nachfolgenden Beschreibung in Verbindung mit der Zeichnung. Es zeigt:
- **Fig. 1**: eine perspektivische Ansicht einer erfindungsgemäßen Atemmaskenanordnung;
- **Fig. 2**: eine weitere perspektivische Ansicht der erfindungsgemäßen Atemmaskenanordnung ohne Schlauchanschlusshülse;
- **Fig. 3**: eine perspektivische Ansicht der Applikationsstruktur der vorangehend gezeigten Atemmaskenanodnung;
- **Fig. 4**: eine perspektivische Ansicht der Applikationsstruktur mit daran angeschlossenem Dockingport;
- **Fig. 5**: eine perspektivische Ansicht eines, aus Trägerabschnitt und Rahmenabschnitt gebildeten Integralteils;
- **Fig. 6**: eine weitere perspektivische Darstellung des Integralteils nach Fig. 6 zur Erläuterung der zur Ankoppelung des Dockingports vorgesehenen Verrastungseinrichtung;
- **Fig. 7**: eine Seitenansicht der erfindungsgemäßen Atemmaskenanordnung;
- **Fig. 8**: eine vereinfachte perspektivische Ansicht einer Stirnauflageeinrichtung mit zwei schwenkbewegbar gelagerten padartigen Auflageelementen sowie andeutungsweise einer Atemmaske;
- **Fig. 9**: eine vereinfachte Schnittansicht zur Erläuterung einer bevorzugten Ausführungsform eines schwenkbewegbar gelagerten Auflageelementes;
- **Fig. 10**: eine Draufsicht auf zwei integral miteinander ausgebildete Auflageelemente, von welchen jedes individuell mit einer Schwenklagerungseinrichtung versehen ist;
- **Fig. 11**: eine weitere Ausführungsform eines Auflageelementes mit einer konkav gewölbten Auflagefläche und einem Kalottenabschnift zur Lagerung des Auflageelementes in schwenkbewegbarer Weise;
- **Fig. 12**: eine Skizze zur Erläuterung einer bevorzugten Flächenpressungsverteilung von einem Mittenbereich des Auflageelementes in ihrem Verlauf von einem Zentrumsbereich des Auflageelementes zu dessen Randbereich hin.

### Ausführliche Beschreibung der Zeichnungen

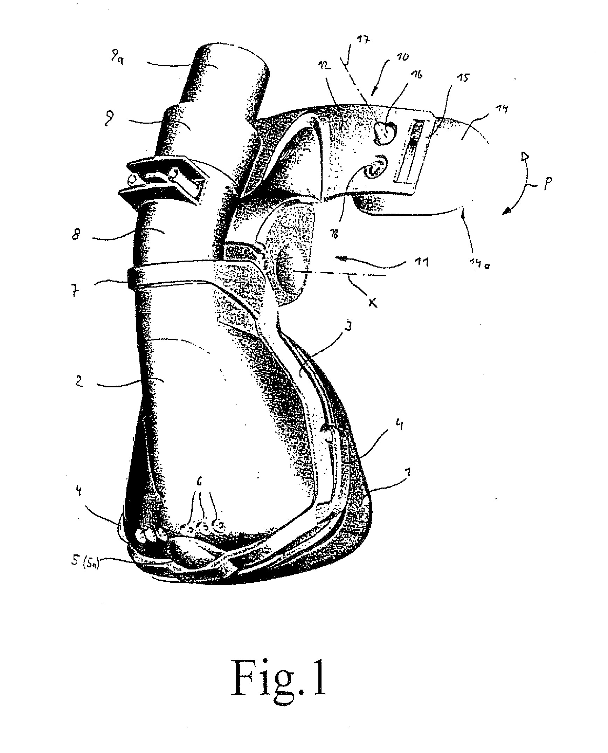

Die in Fig. 1 dargestellte Atemmaskenanordnung umfasst eine, aus einem Elastomermaterial, insbesondere Silikonkautschuk gefertigte Dichtlippeneinrichtung 1 und einen Gewölbekörper 2. Die Dichtlippeneinrichtung 1 ist derart ausgebildet, dass diese eine, zur Aufnahme des Nasenbereichs eines Maskenanwenders vorgesehene Aufnahmeöffnung umsäumt und hierbei vorzugsweise den Nasenrücken sowie den Oberlippenbereich des Maskenanwenders überquert. Die Dichtlippeneinrichtung 1 hat hierbei eine im wesentlichen sattelförmige Silhouette. Die Dichtlippeneinrichtung 1 und der Gewölbekörper 2 sind bei diesem Ausführungsbeispiel einstückig aus einem Elastomermaterial ausgebildet und in einem Rahmenabschnitt 3 aufgenommen.

Der Rahmenabschnitt 3 ist aus einem Kunststoffmaterial gefertigt und weist integral mit diesem, gefertigte Haltebügel 4 auf. Die Haltebügel 4 befinden sich in Applikationsposition der Atemmaskenanordnung auf Wangenhöhe oder auf Höhe der Nasenflügel des Maskenanwenders und ermöglichen die Ankoppelung einer unteren Gurtbandanordnung. Zur zuverlässigen Koppelung des Gewölbekörpers 2 mit dem Rahmenabschnitt 3 ist eine Raststruktur 5 vorgesehen, über welche der Gewölbekörper 2 an dem Rahmenabschnitt 3 in Fügeposition durch Einrasten fixierbar ist. Die Raststruktur umfasst eine, eine Oberseite des Rahmenabschnitts 3 übergreifende Rastnase 5a.

An dem Gewölbekörper 2 sind mehrere Auslassöffnungen 6 ausgebildet, zur Ableitung verbrauchten Atemgases an die Umgebung.

Die Atemmaskenanordnung umfasst weiterhin einen Trägerabschnitt 7, der bei diesem Ausführungsbeispiel integral mit dem Rahmenabschnitt 3 ausgebildet ist.

An dem Trägerabschnitt 7 ist ein hier als Dockingport 8 ausgebildetes Atemgasleitungsorgan lösbar fixiert. Der Dockingport 8 umfasst einen, hier nicht sichtbaren Ringflansch, auf welchen eine Schlauchanschlusshülse 9 drehbewegbar aufgesetzt ist. Die Schlauchanschlusshülse 9 umfasst einen Schlauchanschlussstutzenabschnitt 9a auf den ein Endabschnitt eines Atemgasschlauches aufsteckbar ist.

Die erfindungsgemäße Atemmaskenanordnung umfasst weiterhin eine Stirnabstützeinrichtung 10. die über eine Einstelleinrichtung 11 bewegbar mit dem Rahmenabschnitt 3 gekoppelt ist.

Die Einstelleinrichtung 11 ist derart ausgebildet, dass diese ein Verschwenken der Stirnabstützeinrichtung 10 gegenüber dem Rahmenabschnitt 3 um die hier dargestellte Schwenkachse X ermöglicht. Die Einstelleinrichtung 11 umfasst einen, Fixiermechanismus, durch welchen die Stirnabstützeinrichtung 10 und der Rahmenabschnitt 3 in der gewählten Relativposition fixiert werden können.

Die Stirnabstützeinrichtung 10 umfasst einen Bügelabschnitt 12 an welchem Stirnpolsterelemente 14 anbringbar sind. An dem Bügelabschnitt 12, sind ähnlich wie an dem Rahmenabschnitt 3, Haltebügel 15 ausgebildet, zur Koppelung des Bügelabschnitts 12 mit einer oberen Gurtbandanordnung eines zur Fixierung der Atemmaskenanordnung vorgesehenen Kopfbandes.

Die Stirnpolsterelemente 14 sind aus einem Elastomermaterial gefertigt und bilden auf ihrer, in Applikationsposition dem Maskenanwender zugewandten Unterseite 14a eine Auflagefläche mit einer vorgegebenen Flächenpressungsverteilung. Die Stirnpolsterelemente 14 sind jeweils über einen Steckfuss 16 mit dem Bügelabschnitt 12 gekoppelt. Der Steckfuss 16 ist derart exzentrisch an dem jeweiligen Stirnpolsterelement 14 ausgebildet, dass durch Schwenken der Stirnpolsterelemente 14 wie durch den Pfeil P angedeutet, um die Steckfussachse 17 unterschiedliche Auflagepositionen der Unterseite 14a der Stirnpolsterelemente 14 auf der Stirnfläche des Maskenanwenders erreicht werden können. Durch Auswahl der Schwenkposition des Stirnpolsterelementes 14 sowie durch Auswahl der zur Aufnahme des Steckfusses 16 vorgesehenen Aufnahmeöffnung 18 können unterschiedliche Auflagepositionen erreicht werden. Bei dem hier gezeigten Ausführungsbeispiel sind für das linke und das rechte Stirnpolsterelement 14 jeweils 2 voneinander beabstandete Aufnahmeöffnungen 18 in dem Bügelabschnitt 12 ausgebildet.

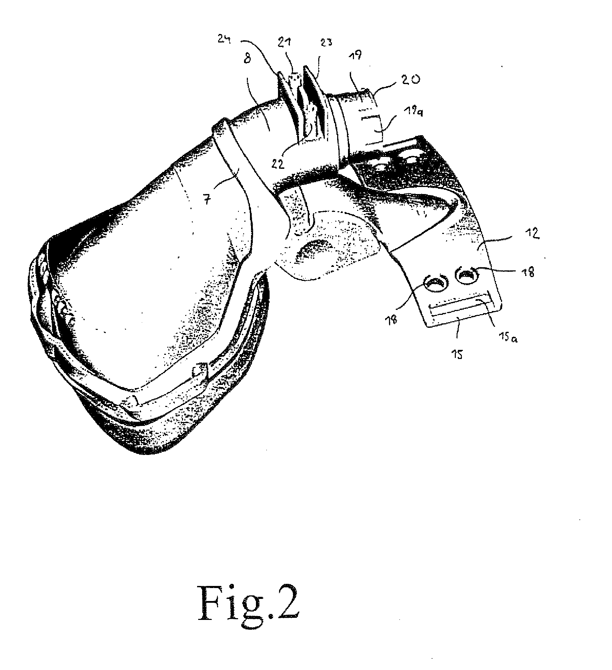

Die Stirnpolsterekemente 14 können durch Herausziehen der Steckfüsse 16 aus den Aufnahmeöffnungen 18 von dem Bügelabschnitt 12 abgenommen werden, wie dies in Fig. 2 gezeigt ist. Die Mittelachsen der Aufnahmeöffnungen 18 sind bei diesem Ausführungsbeispiel um etwa 10 mm voneinander beabstandet. Die Exzentrizität des an dem jeweiligen Stirnpolsterelement 14 ausgebildeten Steckfusses 16 (s. hierzu Fig. 1) beträgt ebenfalls ca. 10 mm. Infolge der exzentrischen Anordnung des Steckfusses 16 sowie der beabstandeten Ausbildung der Aufnahmeöffnung 18, wird im wesentlichen senkrecht zu einer die Augenbrauen des Maskenanwenders verbindenden Linie, eine Höhenveränderbarkeit der Stirnpolsterelemente im Bereich von ca. 30 mm erreicht. In seitlicher Richtung, d.h. in Richtung der genannten, die Augenbrauen verbindenden Linie, ist ebenfalls eine Veränderung der Auflageposition im Bereich von 20 mm möglich. Der zur Ankoppelung einer oberen Gurtbandanordnung vorgesehene Haltebügel 15 ist derart nahe an den Aufnahmeöffnungen 18 angeordnet, dass die durch den Haltebügel begrenzte Durchführungsöffnung 15a zum Maskenanwender hin, durch das Stirnpolsterelement 14 abgedeckt ist.

In der Darstellung gemäß Fig. 2 ist die in Fig. 1 gezeigte Schlauchanschlusshülse 9 von dem Dockingport 8 abgenommen. Erkennbar ist in dieser Ansicht ein integral mit dem Dockingport 8 ausgebildeter Ringflansch 19, der mehrere Zungenelemente 19a aufweist, die zum Zentrum des durch den Dockingport 8 gebildeten Durchgangskanals hin elastisch auslenkbar sind. An den Zungenelementen 19a ist ein Rastwulst 20 ausgebildet der mit einer komplementär an der Schlauchanschlusshülse 9 ausgebildeten Innenumfangsnut, in Eingriff bringbar ist. Die Geometrie des Rastwulstes 20, der in der Drehhülse 9 ausgebildeten Innenumfangsnut sowie die Elastizität der Zungenelemente 19a ist derart abgestimmt, dass die Schlauchanschlusshülse 9 bei Überschreiten einer vorbestimmten Zugkraft werkzeuglos von dem Ringflansch 19 abziehbar ist. Der Ringflansch 19 und die Schlauchanschlusshülse 9 sind weiterhin derart passend ausgebildet, dass die Schlauchanschlusshülse 9 leicht drehbar auf dem Ringflansch 19 sitzt.

Der Dockingport 8 ist bei diesem Ausführungsbeispiel mit Schlauchanschlussstutzen 21, 22 versehen auf die ein Stecker oder Schlauchelement aufsteckbar ist. Die Schlauchanschlussstutzen 21, 22 bilden jeweils einen Durchgangskanal der in den in dem Dockingport 8 gebildeten Atemgaskanal mündet. Die Schlauchanschlussstutzen 21, 22, sind bei Nichtgebrauch durch ein Pfropfen- oder Kappenelement (nicht dargestellt) verschliessbar, das vorzugsweise reibschlüssig auf diesen Schlauchanschlussstutzen 21, 22 sitzt. Bei dieser Ausführungsform sind die Schlauchanschlussstutzen 21, 22 in einer Nut versenkt angeordnet, die durch zwei aufragende Nutwände 23, 24 begrenzt ist. Der Dockingport 8 ist an dem Trägerabschnitt 7 über eine Steckverbindungsstruktur gekoppelt.

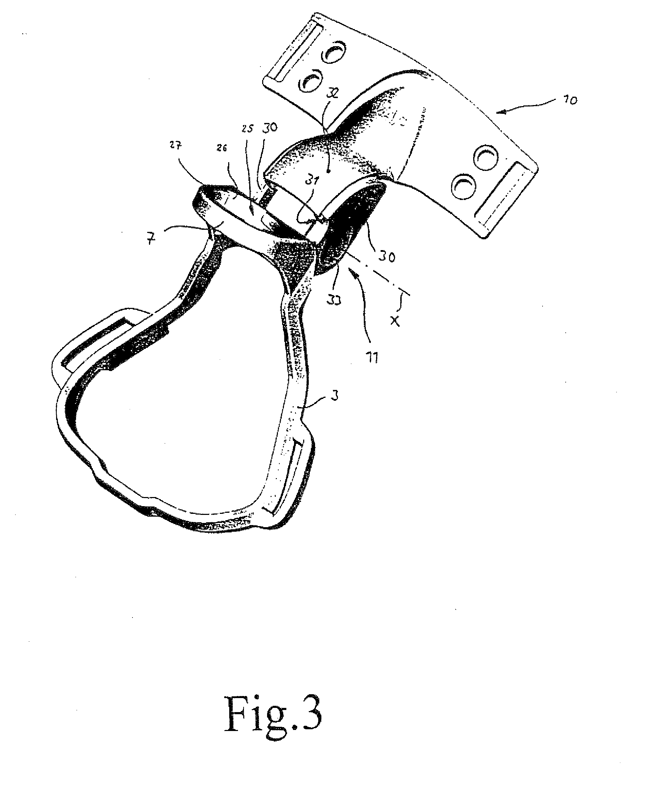

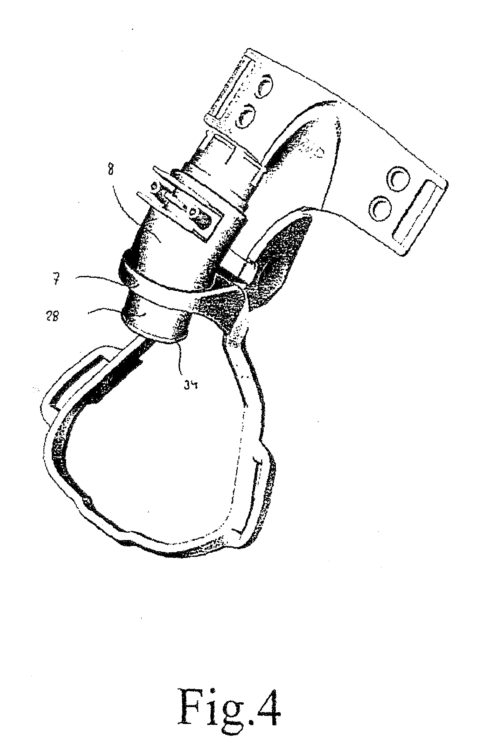

Die Rastverbindungsstruktur zur Koppelung des Dockingports 8 mit dem Trägerabschnitt 7, ist wie aus der Darstellung nach Fig. 3 ersichtlich, bei diesem Ausführungsbeispiel als Drehverrastungs- bzw. als Bajonettverbindungsstruktur 25 ausgebildet. Die Bajonettverbindungsstruktur 25 umfasst zwei einander diametral gegenüberliegende und durch Einsatzfalze 26 voneinander getrennte Rastbrücken 27. Die Rastbrücken 27 geraten in Verriegelungsposition mit zwei Rastvorsprüngen in Eingriff, die an einem Einsteckflanschabschnitt 28 (Fig. 4) des Dockingports ausgebildet sind.

Der Trägerabschnitt 7 ist integral mit dem Rahmenabschnitt 3 ausgebildet und bildet dabei die zur Aufnahme des Einsatzstutzens 28 vorgesehene und partiell von den Rastbrücken 27 umsäumte Einsatzöffnung. Im Übergangsbereich zwischen dem Trägerabschnitt 7 und dem Rahmenabschnitt 3 sind zwei Führungsflanken 30 vorgesehen, die ebenfalls integral mit dem Rahmenabschnitt 3 ausgebildet sind und Teil der Einstelleinrichtung 11 bilden. Die Führungsflanken 30 bilden eine Bogenführung 31 in welcher ein Koppelungsabschnitt 32 der Stirnabstützeinrichtung 10 verschiebbar geführt ist. Die Bogenführung 31 sowie der darin geführte Bereich des Koppelungsabschnitts 32 sind derart ausgebildet, dass die Stirnabschnittseinrichtung 10 und der Rahmenabschnitt 3 zueinander um die bereits in Fig. 1 gezeigte Schwenkachse X bewegbar sind. An den Führungsflanken 30 ist eine Betätigungszone 33 ausgebildet, zur Aufbringung einer die Einstelleinrichtung 11 in eine Freigabestellung bringenden Entriegelungskraft. Die Entriegelungskraft kann insbesondere bei Umgreifen des Dockingports 8, mit dem Daumen und dem Zeigefinger und Auflage der entsprechenden Fingerspitzen, auf der Betätigungszone 33 aufgebracht werden. Alternativ zu der hier vorgesehenen Bogenführung 31, ist es auch möglich, die Einstelleinrichtung 11 derart auszubilden, dass eine Relativbewegung zwischen der Stirnabstützeinrichtung 10 und dem Rahmenabschnitt 3 entlang einer von einer Bogenbahn abweichenden Bewegungsbahn ermöglicht ist. An der Stirnabstützeinrichtung 10 können auch Stirnpolstereinrichtungen angebracht werden, die in ihrem Aufbau von den Stirnpolsterelementen 14 gemäß Fig. 1 abweichen.

In Fig. 4 ist die Applikationsstruktur der erfindungsgemäßen Atemmaskenanordnung in einem Montagezustand dargestellt, bei welchem der erfindungsgemäße Dockingport 8 in die, in dem Trägerabschnitt 7 gebildete Einsatzöffnung eingesetzt - und über die, im Bereich der Einsatzöffnung ausgebildete Bajonettverschlusseinrichtung lagerichtig fixiert ist. Der Dockingport 8 bildet bei dieser Ausführungsform ein rohrkrümmerartiges Leitungsorgan, durch welches das über die Schlauchanschlusshülse 9 (Fig. 1) zufliessende Atemgas um einen Winkel von etwa 30° zur Nasenspitze des Maskenanwenders hin abgelenkt wird. Durch die derartige Zuführung des Atemgases zu dem von dem Gewölbekörper 2 (Fig. 1) begrenzten Maskeninnenraum wird eine vorteilhafte Überströmung des Nasenrückens des Maskenanwenders erreicht.

Durch den modularen Aufbau der erfindungsgemäßen Atemmaskenanordnung wird es möglich, eine dem jeweiligen Anwendungsfall verbessert Rechnung tragende Konfiguration der Atemmaskenanordnung vorzunehmen. Der in den Trägerabschnitt 7 eingesetzte Dockingport 8 weist einen Einsatzstutzen 28 auf, der über den Trägerabschnitt 7 zu dem Rahmenabschnitt 3 hin hervorragt. Auf diesen Einsatzstutzen 28 ist unter geringer elastischer Aufweitung ein Einsatzöffnungsabschnitt des Gewölbekörpers 2 aufsetzbar. An dem Einsatzstutzen 28 ist ein Umfangswulst 34 ausgebildet, durch welchen der Gewölbekörper 2 und der Einsatzstutzen 28 in einer definierten Fügestellung gehalten werden. Dieser Umfangswulst 34 kann mit einer entsprechend in dem Gewölbekörper 2 ausgebildeten Innenumfangsnut in Eingriff treten oder auch auf einer Innenfläche des Gewölbekörpers 2 aufsitzen. Der Trägerabschnitt 7 und der Dockingport 8 sind derart ausgebildet, dass der Dockingport einen, den Nasenwurzelbereich überbrückenden Atemgasleitungsabschnitt bildet. Hierdurch wird eine geringe Beeinträchtigung des Sichtfeldes erreicht.

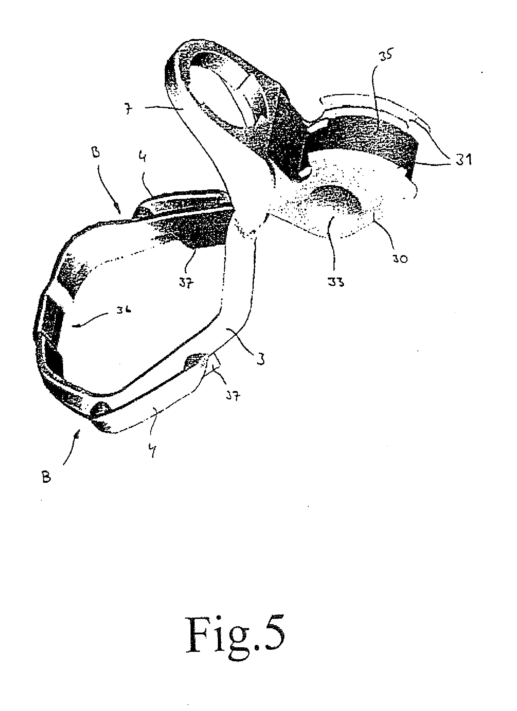

In Fig. 5 ist eine weitere perspektivische Ansicht des den Rahmenabschnitt 3 bildenden Integralteils dargestellt. Die einstückig mit dem Rahmenabschnitt 3 gefertigten Führungsflanken 30, sind derart ausgebildet, dass die Aufbringung einer Lösekraft auf die Betätigungszonen 33 die Führungsflanken 30 im Bereich der Bogenführung 31 auseinanderdrängt. Beim vorliegenden Ausführungsbeispiel wird hierzu durch einen Bogensteg 35 ein Hebel/Kippeffekt erzielt. Die Rückstellung der Führungsflanken 30 im Bereich der Bogenführung 31 erfolgt in Folge der Materialeigenelastizität. Die Fixierwirkung kann im Wege einer reibschlüssigen Klemmung der zwischen den Führungsflanken 30 geführten Kontaktzonen des Koppelungsabschnitts 32 erfolgen. Alternativ hierzu oder in Kombination damit, ist es auch möglich, die Fixierwirkung durch formschlüssige Koppelung, beispielsweise im Wege einer Fein-Verzahnung zu erreichen.

Vorzugsweise ist eine Anschlageinrichtung vorgesehen, die den maximalen Schwenkbereich der Stirnabstützeinrichtung 10 gegenüber dem Rahmenabschnitt 3 begrenzt. Es ist möglich, diese Anschlageinrichtung derart auszubilden, dass diese beispielsweise durch Vergrößern der auf die Betätigungszone 33 aufgebrachten Betätigungskräfte in einen Zustand gelangt in welchem die Stirnabstützeinrichtung 10, von der mit dem Rahmenabschnitt 3 verbundenen Gelenkstruktur, trennbar ist.

In der Darstellung nach Fig. 5 ist weiterhin ein Ausbuchtungsbereich 36 des Rahmenabschnitts 3 erkennbar, durch welchen die Positionierung und Fixierung des Gewölbekörpers 2 in dem Rahmenabschnitt 3 verbessert ist. Die Positionierung des Gewölbekörpers 2 in dem Rahmenabschnitt 3 wird weiterhin unterstützt, durch zwei Fixiersehüde 37 die im Bereich der Haltebügel 4 an der Unterseite des Rahmenabschnitts 3 ausgebildet sind und in Klemmtaschenabschnitte eintauchen, die im Übergangsbereich der Dichtlippeneinrichtung 1 in dem Gewölbekörper 2 ausgebildet sind. Der Rahmenabschnitt 3 weist eine im wesentlichen sattelförmige Silhouette auf und erreicht seine maximale Breite in einer Zone des Rahmenabschnittes 3, die sich in Applikationsposition der Atemmaskenanordnung in etwa auf Höhe der Nasenflügel des Maskenanwenders befindet. Die Haltebügel 4 erstrecken sich von dieser Zone B maximaler Breite zum Augenbereich des Maskenanwenders hin, aufwärts. Die Innenkanten der von den Haltebügeln 4 umsäumten Durchführungsöffnungen sind gerundet ausgebildet, um ein etwaiges Durchscheuern der durch diese Öffnungen hindurchgeführten Bandabschnitte zu vermeiden. In dieser Darstellung ist die zur Fixierung des Dockingports in dem Trägerabschnitt 7 ausgebildete Verrastungseinrichtung nochmals erkennbar.

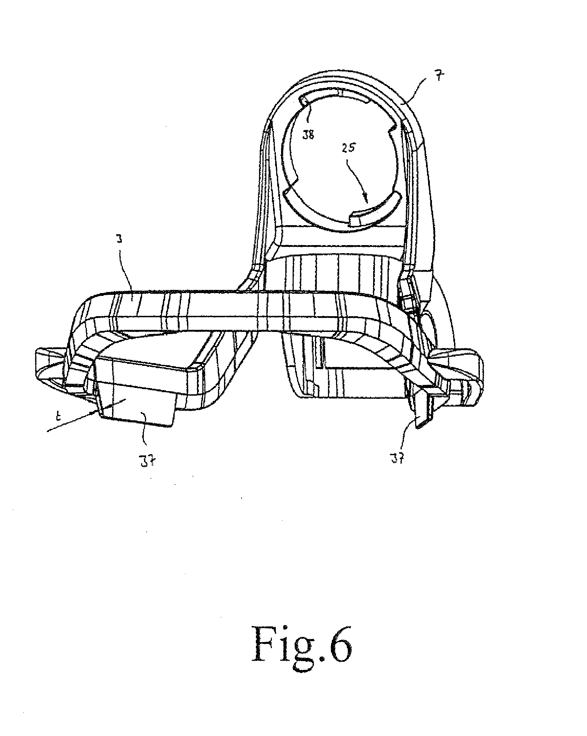

In Fig. 6 sind die an der Unterseite des Rahmenabschnitts 3 ausgebildeten Fixierschilde 37 aus einer anderen Perspektive erkennbar. Die Fixierschilde 37 weisen eine, quer zur Fügerichtung gemessene Dicke t im Bereich von 0,8 bis 3 mm auf. Die Fixierschilde sind in Fügerichtung verjüngt ausgebildet. Die Fixierschilde 37 sowie insbesondere auch der an dem Gewölbekörper 2 anliegende Innenumfangsbereich des Rahmenabschnitts 3 kann mit einer die Koppelung des Gewölbekörpers noch weiter unterstützenden Profilierung versehen sein. Vorzugsweise sind feine, in Umfangsrichtung des Rahmenabschnitts 3 verlaufende Profilrillen an dem Rahmenabschnitt sowie an dem hieran anliegenden Abschnitt des Gewölbekörpers 2 ausgebildet.

Die an dem Trägerabschnitt 7 ausgebildete Bajonettverbindungsstruktur 25 umfasst einen Endanschlag 38, durch welchen die Endposition des Dockingports 8 in Koppelungsstellung festgelegt ist. Die Bajonettverbindungsstruktur 25 ist bei diesem Ausführungsbeispiel derart ausgebildet, dass die maximale Fixierkraft in der durch den Endanschlag 38 festgelegten Endstellung erreicht wird.

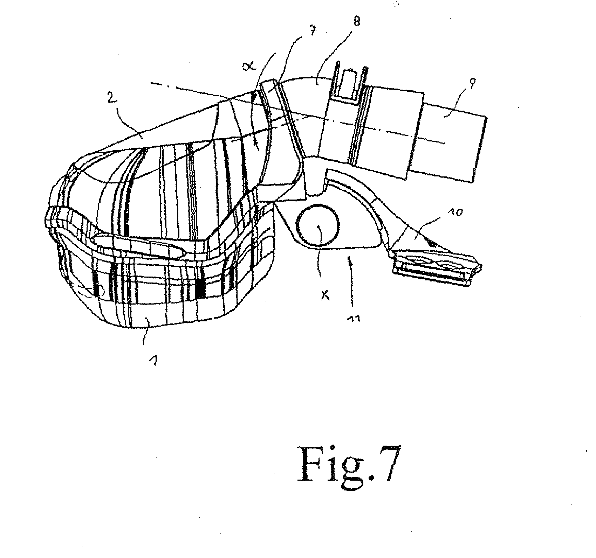

In Fig. 7 ist die erfindungsgemäße Atemmaskenanordnung - mit Ausnahme der Stirnpolsterelemente 14 ( Fig.1) - in vollständig zusammengebauten Zustand aus Richtung der Schwenkachse X gesehen, dargestellt. Wie in dieser Ansicht erkennbar, bildet der erfindungsgemäß lösbar in den Trägerabschnitt 7 einsetzbare Dockingport 8, ein Koppelungselement zur Koppelung des elastomeren Gewölbekörpers 2, mit einer drehbar gelagerten Schlauchanschlusshülse 9, durch welches das zuströmende Atemgas um einen Winkel α abgelenkt wird, der bei diesem Ausführungsbeispiel 32° beträgt.

Der Dockingport 8 kann aus einem mehrere Dockingports 8 enthaltenen Satz ausgewählt sein und bildet ein Schnittstellenteil, durch welches der Gewölbekörper 2 mit unterschiedlichen Schlauchsystemen koppelbar ist. Auch über die auf den Dockingport 8 aufgesetzte Schlauchanschlusshülse 9 kann die Kompatibilität zu unterschiedlichen Atemgasleitungssystemen sichergestellt werden. Es ist möglich, mehrere, jeweils auf bestimmte Gesichtstypen abgestimmte Dichtlippeneinrichtungen vorzusehen und eine bedarfsgerechte Konfiguration der erfindungsgemäßen Atemmaskenanordnung dadurch zu erreichen, dass ein, der individuellen Gesichtstektur des Maskenanwenders besonders gut Rechnung tragendes, aus Dichtlippeneinrichtung 1 und Gewölbekörper 2 bestehendes Elastomerelement, in die erfindungsgemäße Maskenanordnung integriert wird. Es ist auch möglich, mehrere, jedoch zur Einstelleinrichtung 11 kompatible Varianten der Stirnauflageeinrichtung vorzusehen und die erfindungsgemäße Maskenanordnung durch eine, der individuellen Gesichtstektur besonders gut Rechnung tragende Variante der Stirnabstützeinrichtung 10 zu bestücken. Die Stirnabstützeinrichtung 10 kann alternativ zu den in Fig. 1 dargestellten Stirnpolsterelementen auch mit anderweitigen Stirnpoastereinrichtungen zur gepolsterten Auflage auf der Stirn des Maskenanwenders bestückt werden. Es ist möglich, derartige Polstereinrichtungen, beispielsweise in eine obere Stirnbandanordnung zu integrieren und die Stirnabstützeinrichtung, beispielsweise über eine Klettverbindungsstruktur, auf diese gepolsterte Stirnbandanordnung aufzusetzen.

Die Erfindung ist nicht auf das vorangehende Ausführungsbeispiel beschränkt. Beispielsweise ist es auch möglich, in den erfindungsgemäß ausgestalteten Rahmenabschnitt 3 einen Gewölbekörper 2 einzusetzen der nicht aus einem Elastomermaterial gefertigt ist.

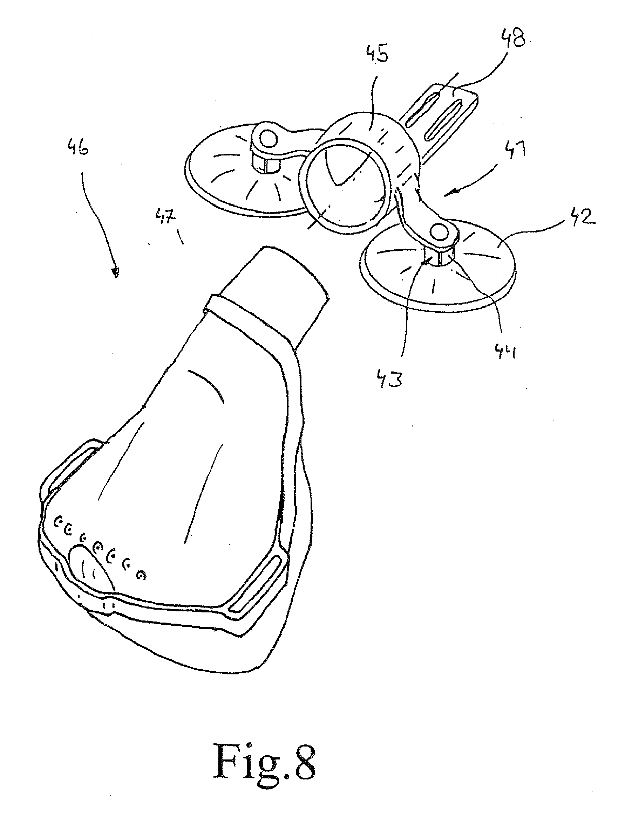

Die in Fig. 8 gezeigte Stirnauflageeinrichtung umfaßt eine Halteeinrichtung 41, zur Halterung eines Auflageelementes 42 in schwenkbewegbarer Weise. Die Halteeinrichtung 41 umfaßt hierzu eine Schwenkhalterung 43, die hier mehrere Fixierelemente 44 aufweist, die Teil einer Kugelgelenkeinrichtung bilden.

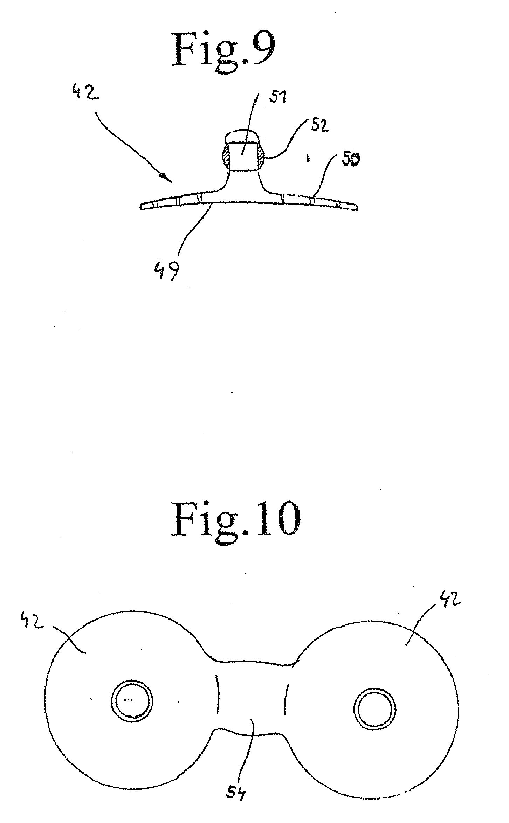

Das Auflageelement 42 weist bei der hier dargestellten Ausführungsform eine tellerartige Gestalt auf und ist aus einem Elastomermaterial, hier volltransparenter Silikonkautschuk gebildet. Das Auflageelement 42 ist über die Fixierelemente 44 um wenigstens zwei Raumachsen kippbar gelagert. Um eine möglichst leichtgängige Bewegbarkeit des Auflageelementes 42 zu gewährleisten, ist zwischen den Fixierelementen 44 und einem Befestigungsschaftabschnitt (nicht sichtbar) des Auflageelementes 42 ein Ringkörper vorgesehen, der eine sphärische Außenfläche aufweist. (Einzelheiten hierzu werden unter Bezugnahme auf Fig. 9 noch ausführlich erläutert.)

Die Halteeinrichtung 41 umfaßt weiterhin einen Koppelungsabschnitt 45 zur Koppelung der Halteeinrichtung 41 mit einer Atemmaske 46.

Bei der hier dargestellten Ausführungsform ist der Koppelungsabschnitt 55 als ringartiges Element ausgebildet, das unmittelbar auf einen entsprechend komplementär ausgebildeten Anschlußstutzen 47 der Atemmaske 46 aufsteckbar ist. Die Halteeinrichtung 1 ist bei der hier dargestellten Ausführungsform mit einem Befestigungsabschnitt 48 versehen und über diesen mit einem Kopfband verbindbar.

In Fig. 9 ist eine bevorzugte Ausführungsform des Auflageelementes 42 dargestellt. Das Auflageelement 42 ist aus einem elastomeren Material gebildet und weist eine schwach konkav gekrümmte Auflagefläche 49 auf. Das Auflageelement 42 ist mit mehreren feinen Durchgangsbohrungen 50 versehen, durch welche ein Druckausgleich des zwischen dem Auflageelement 42 und der Stirn des Patienten ggf. definierten Zwischenraumes mit der Umgebung erreicht werden kann. Hierdurch wird auf vorteilhafte Weise ein Ansaugen des Auflageelementes 42 an dem Stirnbereich des Patienten verhindert.

Das Auflageelement 42 weist bei der hier dargestellten Ausführungsform einen Schaftabschnitt 51 auf. An diesem Schaftabschnitt 51 ist ein Ringelement 52 vorgesehen, das eine sphärische Außenfläche bildet. Im Zusammenspiel mit diesem Ringelement 52 wird eine vergleichsweise leichtgängige Kugelgelenkeinrichtung geschaffen. Alternativ dazu ist es auch möglich, auf das Ringelement zu verzichten und den entsprechenden sphärischen Abschnitt unmittelbar an dem Schaftabschnitt 51 des Auflageelementes 42 auszubilden.

Bei der in Fig. 10 dargestellten Ausführungsform der Erfindung sind zwei Auflageelemente 42 vorgesehen, die über einen integralen Mittelsteg 54 miteinander verbunden sind. Der Mittelsteg 54 ist derart ausgebildet, daß dieser weiterhin ein Schwenken und Verkippen der beiden Auflageelemente 42 zueinander in einem ausreichenden Winkelbereich zuläßt. Der Abstand der Zentren der beiden Auflageelemente 42 voneinander entspricht vorzugsweise in etwa dem Augenabstand des Maskenanwenders.

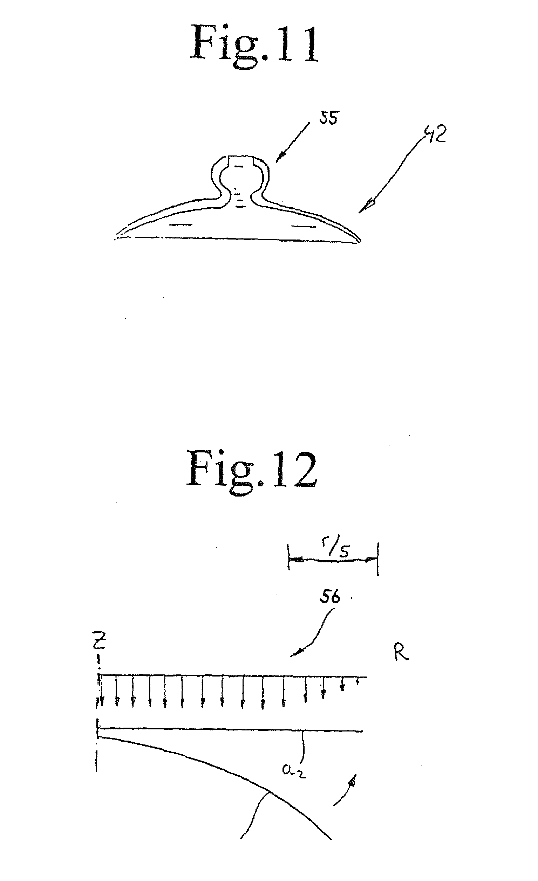

In Fig. 11 ist eine weitere Ausführungsform eines Auflageelementes 42 dargestellt, wobei die Verkippbarkeit des die Auflagefläche bildenden Abschnitts des Auflageelementes über eine Elastomerstruktur 55 erreicht wird, die hier integral mit dem Auflageelement 52 ausgebildet ist. Bei der hier dargestellten Ausführungsform umfaßt die Elastomerstruktur eine im wesentlichen sphärischen Innenraum, der unter vorübergehender elastischer Aufweitung auf einen sphärischen Zapfenabschnitt aufsteckbar ist. Bei der hier dargestellten Ausführungsform weist das Auflageelement ebenfalls einen konkav gewölbten Basiskörper auf, der bei entsprechendem Aufsetzen des Auflageelementes abgeflacht wird.

Die Krümmung des Basiskörpers des Auflageelementes ist vorzugsweise derart gewählt, daß sich beim Aufsetzen auf eine Ebene eine definierte Flächenpressungsverteilung ergibt. Diese Flächenpressungsverteilung ist beispielhaft in Fig. 12 dargestellt. Die untere, gewölbte Linie a1 symbolisiert hierbei die Auflagefläche des Auflageelementes in seiner Ausgangsstellung.

Die hier gerade dargestellte Linie a2 symbolisiert die in Applikationsposition entsprechend deformierte Auflagefläche des Auflageelementes 42. Im Rahmen der Verformung der Auflagefläche des Auflageelementes ergibt sich die hier vereinfacht durch eine Pfeileschar 56 angedeutete Flächenpressungsverteilung. Die Flächenpressungsverteilung ist hier derart gewählt, daß sich vom Zentrum Z ausgehend zum Randbereich hin zunächst eine im wesentlichen gleichmäßige Flächenpressungsverteilung ergibt, wobei im Bereich von R/5 zum Rand R hin die Flächenpressung allmählich abnimmt.

Die Erfindung ist nicht auf die vorangehend beschriebenen Ausführungsbeispiele beschränkt. Beispielsweise ist es auch möglich, die Stirnauflageelemente abweichend von der hier gewählten tellerartigen Gestalt rechteckförmig oder polygonal auszubilden.

## Patentansprüche

1. Atemmaskenanordnung mit einem Gewölbekörper, einer Dichtlippeneinrichtung zur Auflage auf der Gesichtsfläche eines Maskenanwenders, einer Atemgasleitungseinrichtung zur Zuleitung von Atemgas zu einem von dem Gewölbekörper begrenzten und mit der Nasen- und/oder Mundöffnung des Maskenanwenders in Verbindung stehenden Maskeninnenraum und einer Applikationsstruktur zur Applikation der Dichtlippeneinrichtung gemeinsam mit dem Gewölbekörper, wobei die Applikationsstruktur einen Trägerab- schnitt aufweist an welchem ein Atemgasleitungsorgan lösbar angekoppelt ist.

2. Atemmaskenanordnung nach Anspruch 1, **dadurch gekennzeichnet, dass** das Atemgasleitungsorgan als Rohrstutzen ausgebildet ist.

3. Atemmaskenanordnung nach Anspruche 1 oder 2, **dadurch gekennzeichnet, dass** die Applikationsstruktur einen Rahmenabschnitt umfasst.

4. Atemmaskenanordnung nach wenigstens einem der Ansprüche 1 bis 3, **dadurch gekennzeichnet, dass** der Rahmenabschnitt integral mit dem Trägerabschnitt ausgebildet ist.

5. Atemmaskenanordnung nach wenigstens einem der Ansprüche 1 bis 4, **dadurch gekennzeichnet, dass** der Trägerabschnitt eine Einsatzöffnung aufweist und das Atemgasleitungsorgan lösbar in diese Einsatzöffnung einsetzbar ist.

6. Atemmaskenanordnung nach wenigstens einem der Ansprüche 1 bis 5, **dadurch gekennzeichnet, dass** eine Verrastungseinrichtung vorgesehen ist, zur Fixierung des Atemgasleitungsorgans an dem Trägerabschnitt.

7. Atemmaskenanordnung nach wenigstens einem der Ansprüche 1 bis 6, **dadurch gekennzeichnet, dass** der Gewölbekörper mit Öffnungen versehen ist, zur Ableitung von CO2 befrachtetem Atemgas.

8. Atemmaskenanordnung nach wenigstens einem der Ansprüche 1 bis 7, **dadurch gekennzeichnet, dass** die Applikationsstruktur eine Stirnabstützeinrichtung umfasst, zur Abstützung der Anordnung im Stirnbereich.

9. Atemmaskenanordnung nach wenigstens einem der Ansprüche 1 bis 8, **dadurch gekennzeichnet, dass** eine Gelenkvorrichtung vorgesehen ist und die Stirnabstützeinrichtung mit dem Rahmenabschnitt (3) schwenkbewegbar gekoppelt ist, wobei die Stirnabstützeinrichtung vorzugsweise über eine Einstelleinrichtung bewegbar mit dem Rahmenabschnitt verbunden ist, die einen Fixiermechanismus umfasst, durch welchen die Stirnabstützeinrichtung und der Rahmenabschnitt in der gewählten Relativposition fixiert werden können.

10. Atemmaskenanordnung nach wenigstens einem der Ansprüche 1 bis 9, **dadurch gekennzeichnet, dass** die Gelenkeinrichtung eine Bogenbahnführung umfasst und die Stirnabstützeinrichtung einen Führungsabschnitt aufweist.

11. Atemmaskenanordnung nach wenigstens einem der Ansprüche 1 bis 9, **dadurch gekennzeichnet, dass** der Rahmenabschnitt mit der Dichtlippeneinrichtung lösbar koppelbar ist.

12. Atemmaskenanordnung nach wenigstens einem der Ansprüche 7 bis 11, **dadurch gekennzeichnet, dass** die Stirnabstützeinrichtung mir einer Stirnpolstereinrichtung versehen ist, die vorzugsweise durch pad-artig ausgebildete Elastomerelemente gebildet ist, die über eine Steckverbindungsstruktur mir dem Aufnahmeabschnitt der Stirnstützeinrichtung koppelbar sind.

13. Atemmaskenanordnung nach Anspruch 12, **dadurch gekennzeichnet, dass** die Elastomerelemente auf einer ihrer Auflageseite abgewandten Rückseite mir einem Exzentrisch angeordneten Steckfuß versehen sind, der eine Kippbewegung des am Maskenanwender aufsitzenden Auflageabschnitts zulässt.

14. Atemmaskenanordnung nach wenigstens einem der Ansprüche 9 bis 13, **dadurch gekennzeichnet, dass** die Stirnauflageeinrichtung mehrere, vorzugsweise zwei, miteinander benachbart angeordnete Auflageelemente aufweist, die vorzugsweise über eine flexible Stegeinrichtung miteinander verbunden sind.

15. Atemmaskenanordnung nach wenigstens einem der Ansprüche 9 bis 14, **dadurch gekennzeichnet, dass** die Stirnauflageeinrichtung mit Koppelungsabschnitten versehen ist, welche eine Koppelung der Stirnabstützeinrichtung mit einer oberen Stirnbandanordnung eines Kopfbandes ermöglicht.
